# FASCICULE DE BREVET EUROPEEN

(11) **EP 3 463 585 B1**
(45) Date de publication et mention de la délivrance du brevet: **08.07.2020**
(21) Numéro de dépôt: 17730885.5
(22) Date de dépôt: 01.06.2017
(51) Int. Cl.: A61Q 13/00, A61K 8/06, A61K 8/34, A61K 8/49

(54) **PARFUMS SOUS FORME DE MICROÉMULSIONS AQUEUSES**
DUFTSTOFFE IN FORM VON WÄSSRIGEN MIKROEMULSIONEN
PERFUMES IN THE FORM OF AQUEOUS MICROEMULSIONS

(30) Priorité: 01.06.2016 FR 1654983
(43) Date de publication de la demande: 10.04.2019
(73) Titulaire: Chanel Parfums Beauté, 92200 Neuilly-sur-Seine (FR); Université de Lille, 59800 Lille (FR)
(72) Inventeur: BOUCENNA VERDIER, Oriana, 93320 Les Pavillons Sous Bois (FR); MAHE, Christian, 92200 Neuilly sur Seine (FR); RATAJ-NARDELLO, Véronique, 59710 Pont A Marcq (FR); AUBRY, Jean-Marie, 62590 Oignies (FR); NOLLET, Maxime, 69003 Lyon (FR); LEBEUF, Raphaël, 59000 Lille (FR)
(74) Mandataire: Plasseraud IP
(86) Numéro de dépôt international: PCT/FR2017/051373
(87) Numéro de publication internationale: WO 2017/207935

(56) Documents cités:
- FR-A1- 2 998 476
- US-A1- 2009 130 029
- JOSÉ I. GARCÍA ET AL: "Green solvents from glycerol. Synthesis and physico-chemical properties of alkyl glycerol ethers", GREEN CHEMISTRY, vol. 12, no. 3, 1 janvier 2010 (2010-01-01), page 426, XP055025451, ISSN: 1463-9262, DOI: 10.1039/b923631g

## Description

L'invention concerne des microémulsions aqueuses volatiles parfumantes basées sur l'utilisation de solvo-surfactifs.

Classiquement, les parfums comprennent comme solvants des alcools, tels que l'éthanol ou encore l'isopropanol. L'utilisation de ces solvants présente, cependant, un certain nombre d'inconvénients: ils sont très volatils et inflammables, ce qui entraîne une certaine dangerosité lors de leur production, et dans une certaine mesure, de leur utilisation. D'autre part, leur propre odeur peut interférer avec celle du parfum. En outre, appliqués sur la peau ou les cheveux, ces parfums peuvent entraîner un dessèchement, en particulier chez les consommateurs à peau sensible.

On assiste donc actuellement, pour des raisons notamment de santé publique et/ou d'écologie, à l'émergence de nouvelles compositions. L'objectif poursuivi est la diminution voire l'élimination des composés organiques volatils (tels que les alcools) contenus dans les parfums, en développant des compositions parfumantes sous forme de solutions ou de dispersions aqueuses stables.

Cependant, la plupart des molécules parfumantes sont hydrophobes et ne sont donc pas solubles dans l'eau. Pour contourner ce problème, on doit utiliser des tensioactifs permettant de solubiliser les molécules parfumantes à l'intérieur de micelles formant des microémulsions. Il est souhaitable que les micelles gonflées contenant les parfums soient de petite taille pour que la composition parfumante ait un aspect transparent ou tout au moins translucide. Ainsi, la production de microémulsions répondant à ce critère d'aspect transparent revêt un intérêt majeur.

D'autres contraintes sont liées à la stabilité thermodynamique de la microémulsion, au caractère non-collant de celle-ci et à l'absence de résidu sur la peau ou sur les vêtements. Il est donc important de pouvoir les préparer en utilisant le moins de tensioactif possible.

Il existe donc un besoin pour une composition parfumante stable, contenant une quantité importante de parfum, qui soit transparente ou tout au moins translucide, et qui contienne le moins de tensioactif possible.

La présente invention a pour but de fournir des microémulsions aqueuses, transparentes, substantiellement exemptes d'éthanol, contenant au moins une substance hydrophobe parfumante (de préférence au moins 3% et préférentiellement environ 10% de parfum), et au moins un solvo-surfactif volatil. De telles microémulsions odoriférantes ou parfumantes sont stables, et comprennent le moins possible de substances provoquant des effets indésirables, en particulier sur la peau et/ou sur l'environnement.

Selon l'invention, une substance est « volatile » lorsque sa température d'ébullition est inférieure à 250°C à pression atmosphérique. Les composés « non volatils » ont une température d'ébullition supérieure à 250°C à pression atmosphérique.

La présente invention se rapporte donc à une microémulsion de type huile-dans-eau comprenant, de préférence consistant en, en poids par rapport au poids total de microémulsion :
- 70% à 94% d'eau,
- 1 % à 15% d'au moins une substance hydrophobe parfumante,
- 4% à 20% d'au moins un solvo-surfactif de préférence volatil, et
- 0,1% à 15%, de préférence 1% à 13%, d'au moins un agent hydrotrope ou d'au moins un tensioactif choisi parmi les tensioactifs anioniques, les tensioactifs cationiques, les tensioactifs amphotères et les tensioactifs non ioniques,
   ledit solvo-surfactif étant choisi parmi les dérivés de glycérol monoalkylé de formule (I) suivante : 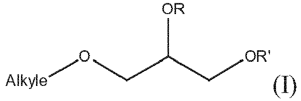 dans lesquels le groupe « Alkyle » est un groupe alkyle linéaire ou ramifié comprenant de 1 à 8 atomes de carbone, et R et R' sont chacun indépendamment H ou un groupe alkyle linéaire ou ramifié comprenant de 1 à 5 atomes de carbone, de préférence un groupe méthyle ou éthyle, à la condition que R est différent de R',
   et leurs mélanges.

La présente invention se rapporte aussi à une microémulsion de type huile-dans-eau comprenant, de préférence consistant en, en poids par rapport au poids total de microémulsion :
- 70% à 94% d'eau,
- 1% à 15% d'au moins une substance hydrophobe parfumante,
- 4% à 20% d'au moins un solvo-surfactif de préférence volatil, et
- 0,1% à 15%, de préférence 1% à 13%, d'au moins un agent hydrotrope ou d'au moins un tensioactif choisi parmi les tensioactifs anioniques, les tensioactifs cationiques, les tensioactifs amphotères et les tensioactifs non ioniques,
   ledit solvo-surfactif étant choisi parmi les mélanges entre i) un composé d'isosorbide obtenu sous les 2 formes endo et exo suivantes: 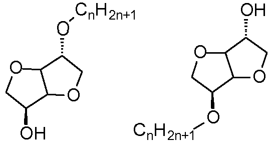 dans lesquelles n = 4 ou 5,
   et ii) un dérivé de glycérol monoalkylé de formule (I) suivante : 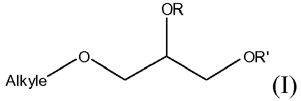 dans laquelle le groupe « Alkyle » est un groupe alkyle linéaire ou ramifié comprenant de 1 à 8 atomes de carbone, et R et R' sont chacun indépendamment H ou un groupe alkyle linéaire ou ramifié comprenant de 1 à 5 atomes de carbone, de préférence un groupe méthyle ou éthyle, à la condition que R est différent de R'.

Optionnellement, la microémulsion selon l'invention comprend de 0.01% à 2% en poids d'au moins un conservateur.

De préférence, la microémulsion selon l'invention est substantiellement exempte d'éthanol, i.e. elle comprend moins de 3% en poids d'éthanol, de préférence moins de 2% en poids d'éthanol, de préférence moins de 1% en poids d'éthanol. Plus préférentiellement, elle est dépourvue d'éthanol, i.e. elle contient 0% en poids d'éthanol.

De manière préférée, les dérivés de glycérol monoalkylé sont les dérivés de formule (I) suivante : 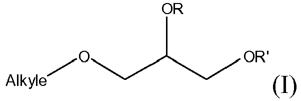 dans lesquels le groupe « Alkyle » est un groupe alkyle linéaire ou ramifié comprenant de 1 à 6 atomes de carbone, et R et R' sont chacun indépendamment H ou un groupe alkyle linéaire ou ramifié comprenant de 1 à 5 atomes de carbone, de préférence un groupe méthyle ou éthyle, à la condition que R est différent de R'.

De manière préférée, les dérivés de glycérol monoalkylé sont les dérivés de formule (I) suivante : 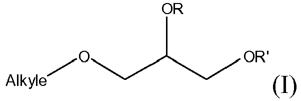 dans lesquels le groupe « Alkyle » est un groupe alkyle linéaire ou ramifié comprenant 3, 4 ou 5 atomes de carbone, et R et R' sont chacun indépendamment H ou un alkyle linéaire ou ramifié comprenant de 1 à 5 atomes de carbone, de préférence un groupe méthyle ou éthyle, à la condition que R est différent de R'.

Plus préférentiellement, les dérivés de glycérol monoalkylés sont les dérivés de formule (I) suivante : 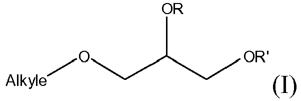 dans lesquels le groupe « Alkyle » est un groupe alkyle linéaire ou ramifié comprenant 3, 4 ou 5 atomes de carbone, R est un groupe méthyle, et R' est H.

Par « microémulsion de type huile-dans-eau », on désigne un système liquide dans lequel une phase huileuse (ou hydrophobe) est dispersée dans une phase continue aqueuse (ou hydrophile), de sorte à former des gouttes de diamètre inférieur à 100 nm. L'interface huile/eau est stabilisée par des composés tensioactifs. De préférence, les gouttes ont un diamètre compris entre 2 et 100 nm.

Ces microémulsions présentent des gouttes qui sont invisibles à l'œil nu et au microscope optique. Elles sont transparentes, tout au moins translucides, contrairement aux émulsions, ce qui est une propriété recherchée notamment pour les compositions parfumantes.

Par « substance hydrophobe », on désigne un corps pur ou un mélange insoluble ou très peu soluble dans l'eau, par nature. Une méthode possible pour déterminer l'hydrophobicité des substances est de mesurer leur solubilité dans différents solvants ou le temps de rétention sur colonne chromatographique (par chromatographie en phase liquide à haute performance HPLC) de ladite substance hydrophobe.

Les substances hydrophobes selon l'invention sont parfumantes, i.e. elles sont odoriférantes et peuvent être utilisées dans les parfums. Par « substance odoriférante » on entend une substance détectable olfactivement par un sujet et/ou par olfactométrie, selon des principes connus de l'homme du métier. Un exemple de méthode permettant la détection d'une substance odoriférante est décrit dans le document EP 0003088. D'autres techniques de détection d'une substance odoriférante sont applicables comme les techniques de chromatographie en phase gazeuse, de spectroscopie de masse ou encore d'analyse par absorption infrarouge. On entend aussi par substance odoriférante une substance qui répand une odeur, de préférence agréable pour au moins 20% de personnes, en particulier un parfum. De préférence, la substance hydrophobe parfumante est une substance hydrophobe parfumante naturelle ou synthétique, plus préférentiellement naturelle. Plus préférentiellement, elle est choisie parmi les terpènes, les huiles essentielles et les composés naturels présentant des propriétés odoriférantes (terpénoïdes), notamment choisis parmi les aldéhydes, les esters, les cétones, les alcools, les phénols, les alcènes et les éthers.

Par "terpènes", on désigne des hydrocarbures dont l'élément de base est l'isoprène, leur formule brute comportant un nombre de carbones multiple de 5, en particulier des terpènes contenant notamment 10 ou 15 atomes de carbone, utilisés en parfumerie.

Par "terpénoïdes", on désigne des dérivés des terpènes, par exemple des alcools, des phénols, des cétones, des aldéhydes, des esters ou des éthers.

Terpènes et terpénoïdes sont contenus dans les "huiles essentielles", désignant le liquide concentré, très souvent odoriférant, volatil, produit par les plantes. Les huiles essentielles sont le plus souvent extraites des organes de la plante par hydrodistillation notamment, mais les constituants de ces huiles sont largement synthétisés par l'industrie.

On peut notamment utiliser les substances hydrophobes parfumantes naturelles suivantes : terpènes: pinènes, camphènes, limonène, cadinène, carène, caryophyllène, alcools : linalol, géraniol, menthol, citronellol,
cétones: menthone, carvone, béta-ionone, thuyone, camphre, cyclopentadécanone,
aldéhydes : citral, citrannal, citronellal, aldéhyde cinnamique, lilial,
esters: acétate de linalyle, acétate de menthyle, acétate de géranyle, succinate de géranyle, phénols : thymol, carvacrol, eugénol, isoeugénol,
éthers: anéthol, eucalyptol, cinéol, oxyde de rose,
alcènes : limonène.

Les huiles essentielles peuvent être les huiles d'ylang-ylang, de bergamote, d'eucalyptus, de lavande, de lavandin, de lemongrass, de patchouli, de menthe poivrée, de pin, de rose, de coriandre, de Shiu, de sauge, de géranium, de palmarosa, de LitseaCubeba, de citron, de citronnelle, de fleur d'oranger, de pamplemousse, de lime, de mandarine, de tangerine, d'orange, de cajeput, de camphre, de romarin, d'anis vert, d'anis étoilé, de fenouil, de basilic, d'estragon, de girofle, de piment, de thym, de sassafras, d'absinthe, d'armoise, de cèdre, d'hysope, de tagetes, de rue, d'élémi, de galbanum, de baies de genièvre, de cabreuva, de bois de gaiac, de bois de santal, de vétiver, d'ambrette, d'angélique, de rhizome d'iris, de carotte, de céleri, de cumin, de livèche, de persil, de cannelle, de cardamome, de gingembre, de noix de muscade, de poivre, d'oliban, de myrrhe, de baume du Pérou, de styrax, de buchu, de camomille ou de ciste (Jean Garnero, "Huiles essentielles", Techniques de l'ingénieur, Traité constantes physico-chimiques, K-345).

La quantité de substance hydrophobe parfumante dans les microémulsions selon l'invention est comprise entre 1% et 15% en poids, de préférence entre 5% et 12% en poids par rapport au poids total de microémulsion.

Par « solvo-surfactif », on désigne un composé amphiphile, cumulant certaines propriétés des tensioactifs, notamment la diminution des tensions superficielle eau/air et interfaciales huile/eau, la capacité d'auto-association dans l'eau, et certaines propriétés des solvants, notamment la capacité à s'évaporer sans laisser de résidus.

Le solvo-surfactif est volatil et est choisi parmi les dérivés de glycérol monoalkylé de formule (I) suivante : 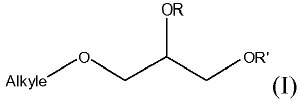 dans lesquels le groupe « Alkyle » est un groupe alkyle linéaire ou ramifié comprenant 1 à 8 atomes de carbone, et R et R' sont chacun indépendamment H ou un groupe alkyle linéaire ou ramifié comprenant de 1 à 5 atomes de carbone, de préférence un groupe méthyle ou éthyle, à la condition que R est différent de R'.

Plus préférentiellement, le solvo-surfactif est le dérivé de glycérol monoalkylé de formule (I) dans laquelle le groupe « Alkyle » est un groupe alkyle linéaire comprenant 3, 4 ou 5 atomes de carbone, et R et R' sont chacun indépendamment H ou un groupe méthyle ou éthyle, à la condition que R est différent de R'.

Plus préférentiellement, le solvo-surfactif est le dérivé de glycérol monoalkylé de formule (I) dans laquelle le groupe « Alkyle » est un groupe alkyle linéaire comprenant 3, 4 ou 5 atomes de carbone, R est un groupe méthyle ou éthyle et R' est H.

Le dérivé de glycérol monoalkylé de formule (I) dans laquelle le groupe « Alkyle » est un groupe alkyle linéaire comprenant 3 atomes de carbone (groupe propyle), R est H et R' est méthyle, est le 1-méthoxy-3-propoxypropan-2-ol. Il est appelé « C301 » dans les exemples de la présente demande.

Le dérivé de glycérol monoalkylé de formule (I) dans laquelle le groupe « Alkyle » est un groupe alkyle linéaire comprenant 3 atomes de carbone (groupe propyle), R est méthyle et R' est H, est le 2-méthoxy-3-propoxypropan-1-ol. Il est appelé « C310 » dans les exemples de la présente demande.

Le dérivé de glycérol monoalkylé de formule (I) dans laquelle le groupe « Alkyle » est un groupe alkyle linéaire comprenant 4 atomes de carbone (groupe butyle), R est H et R' est méthyle, est le 1-méthoxy-3-butoxypropan-2-ol. Il est appelé « C401 » dans les exemples de la présente demande.

Le dérivé de glycérol monoalkylé de formule (I) dans laquelle le groupe « Alkyle » est un groupe alkyle linéaire comprenant 4 atomes de carbone (groupe butyle), R est méthyle et R' est H, est le 2-méthoxy-3-butoxypropan-1-ol. Il est appelé « C410 » dans les exemples de la présente demande.

Le dérivé de glycérol monoalkylé de formule (I) dans laquelle le groupe « Alkyle » est un groupe alkyle linéaire comprenant 5 atomes de carbone (groupe pentyle), R est H et R' est méthyle, est le 1-méthoxy-3-pentoxypropan-2-ol. Il est appelé « C501 » dans les exemples de la présente demande.

Le dérivé de glycérol monoalkylé de formule (I) dans laquelle le groupe « Alkyle » est un groupe alkyle linéaire comprenant 5 atomes de carbone (groupe pentyle), R est méthyle et R' est H, est le 2-méthoxy-3-pentoxypropan-1-ol. Il est appelé « C510 » dans les exemples de la présente demande.

La quantité de solvosurfactif(s) volatil(s) dans la microémulsion selon l'invention est comprise entre 5% et 20% en poids, de préférence entre 7% et 18% en poids.

Les microémulsions selon l'invention ont une qualité de fragrance stable dans le temps correspondant à la durée de vie standard d'un produit cosmétique, et stable en température de 5 à 45°C ou de 15 à 45°C, ce qui correspond aux températures d'exposition et d'usage d'un produit cosmétique.

Les critères techniques de la qualité d'une fragrance, dans le cas d'une fragrance aqueuse, sont :
- la capacité d'une composition parfumée à maintenir, après application, un seuil de perception olfactif dans le temps,
- la capacité d'une composition à maintenir, une fois appliquée, sa forme olfactive dans le temps,
- la capacité d'une composition à ne pas subir d'altérations endogènes ou exogènes, qui pourraient modifier sa forme olfactive, et
- l'innocuité, qui est la faculté d'une composition à ne pas produire d'effets indésirables une fois appliquée sur la peau de l'utilisateur.

Par « agent hydrotrope », on entend un composé amphiphile, comprenant des groupes fonctionnels hydrophiles, utilisé pour permettre la solubilisation de substances faiblement solubles dans une solution aqueuse. Ils permettent d'abaisser la température de point trouble. L'agent hydrotrope est notamment choisi parmi les acides aryl sulfoniques et les alkyl glucosides.

Parmi les alkyl glucosides utilisables, on peut citer l'heptyl glucoside, l'octyl glucoside ou le décyl glucoside. De préférence, l'agent hydrotrope est l'heptyl glucoside.

Par « tensioactif », on désigne un composé de nature amphiphile, non volatil, comportant une partie hydrophile et polaire, et une partie hydrophobe apolaire. Un tensioactif abaisse la tension superficielle des solutions aqueuses et diminue la tension interfaciale entre l'eau et un liquide organique non miscible. Il permet ainsi de solubiliser deux phases non miscibles, telles que l'eau et l'huile, en interagissant par sa partie polaire avec l'eau et par sa partie apolaire avec l'huile. Le tensioactif selon l'invention est choisi parmi les quatre catégories de tensioactifs:
- anioniques: la partie hydrophile est chargée négativement,
- non ioniques : le composé ne comporte aucune charge,
- amphotères ou zwitterioniques: la partie hydrophile comporte une charge positive et une charge négative, la charge globale étant nulle. Le tensioactif zwitterionique est chargé en permanence, tandis que le tensioactif amphotère s'ionise en fonction du pH, et
- cationiques: la partie hydrophile est chargée positivement.

La quantité de tensioactif(s) dans les microémulsions selon l'invention est comprise entre 1% et 10% en poids, de préférence entre 1% et 5% en poids par rapport au poids total de microémulsion.

De façon avantageuse, la microémulsion de l'invention contient au moins un tensioactif anionique, de préférence choisi parmi:
- les alkylsulfonates, notamment le sulfosuccinate de dihexyle (DHS) de formule 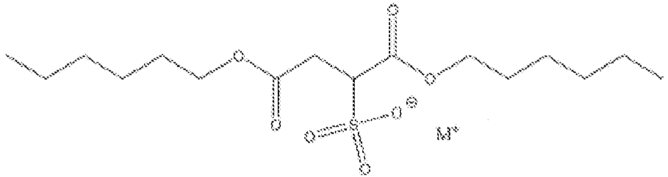 dans laquelle
   M⁺ représente Na⁺, K⁺, NH₄⁺, (HOCH₂CH₂)₃NH⁺, ou notamment le sulfosuccinate d'éthyl-2-hexyle (Aérosol OT® ou AOT de chez CYTEC) de formule 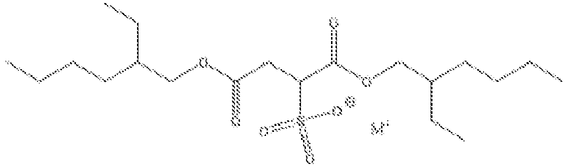 dans laquelle
   M⁺ représente Na⁺, K⁺, NH₄⁺, (HOCH₂CH₂)₃NH⁺,
   - les alkylarylsulfonates de formule 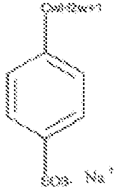 dans laquelle w est un entier compris de 8 à 12,
      en particulier l'isooctyl, l'isononyl et notamment l'isododécylbenzènesulfonate de sodium (SDBS) de formule 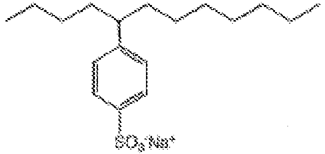
   - les propoxysulfates, notamment les composés alfoterra® de formule 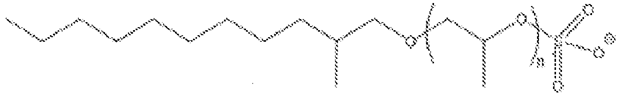 dans laquelle le nombre de motifs propoxylate n est compris de 4 à 8,
      - n pouvant être notamment égal à 4 (alfoterra® 4S, Alf4S),
      - n pouvant être notamment égal à 8 (alfoterra® 8S, Alf8S),
   - les alkylsulfates, notamment les sels du sulfate de lauryle tel que le dodécyl sulfate de sodium (SDS) et les alkyléther sulfates de sodium tel que le lauryléther (laureth) sulfate de sodium (LES),
   - et les sels d'acides gras de formule R-CO₂⁻ M⁺, dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺, notamment le sel d'acide oléique de formule CH₃(CH2)₇CH=CH(CH₂)₇CO₂⁻ M⁺ dans lequel M⁺ a les significations définies ci-dessus.

De préférence, la microémulsion de l'invention comprend au moins un tensioactif anionique choisi parmi le dodécylsulfate de sodium et le laureth sulfate de sodium.

D'une autre façon avantageuse, la microémulsion de l'invention contient au moins un tensioactif non ionique choisi parmi:
- les alcanolamides, notamment le monoéthanolamide (cocamide, MEA) de formule 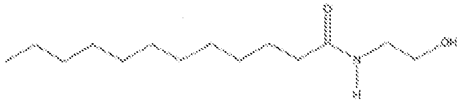 sous réserve que ledit alcanolamide ne soit pas un polyol,
- les alkylpolyglycosides (APG),
- les éthers de polyglycérol,
- et les esters de polyglycérol.

D'une autre façon avantageuse, la microémulsion de l'invention contient au moins un tensioactif amphotère choisi parmi:
- les bétaïnes, notamment la bétaïne de cocamidopropyle (CAPB) de formule 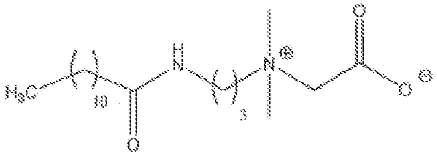
- et les oxydes d'amine, notamment l'oxyde de lauryldiméthylamine de formule

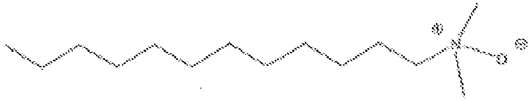

Alternativement, la microémulsion de l'invention contient au moins un tensioactif cationique, de préférence choisi parmi:
- des sels de tétraalkylammonium, notamment des sels de dialkyldiméthylammonium de formule 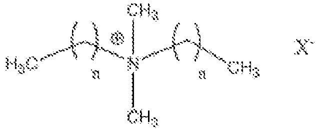 dans laquelle
   X⁻ représente un anion choisi parmi Cl⁻ , Br⁻, I⁻, CH₃COO⁻ ou lactate,
   n est le nombre de méthylène compris de 6 à 12,
   de préférence un sel de didécyldiméthylammoniumde formule 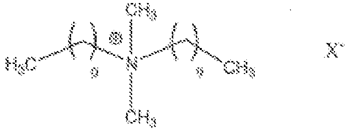 dans laquelle X⁻ a les significations désignées ci-dessus,
- des sels de trialkylammonium, en particulier des halogénures de trialkylammonium, notamment le bromure de dodécyltriméthylammonium (DTAB) de formule H₃C(CH₂)₁₁N⁺(CH3)3,
- des sels de pyridinium, notamment le sel de cétylpyridinium de formule 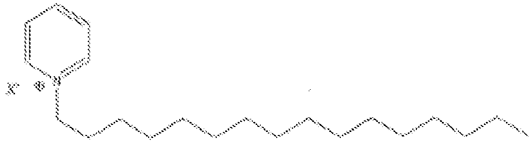 dans laquelle
   X⁻ a les significations désignées ci-dessus,
- des sels de benzalkonium, notamment des sels de formule 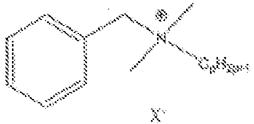 dans laquelle
   X⁻ a les significations désignées ci-dessus,
   p est compris de 8 à 18,
- des sels d'ammonium de la triéthanolamine de formule
   (HO-CH₂-CH₂)₃NH⁺ X- dans laquelle X⁻ a les significations désignées ci-dessus.

De façon très avantageuse, les microémulsions de l'invention sont stables thermodynamiquement et ont un aspect transparent ou translucide et de façon encore plus avantageuse, les microémulsions de l'invention sont stables thermodynamiquement et ont un aspect transparent ou translucide pendant au moins un ou deux ans.

De préférence, la microémulsion selon l'invention consiste en :
- 70% à 90% d'eau,
- 5% à 12% d'au moins une substance hydrophobe parfumante,
- 4% à 18% d'au moins un solvo-surfactif volatil, et
- 0,1% à 10%, de préférence 1% à 5%, d'au moins un agent hydrotrope ou d'au moins un tensioactif choisi parmi les tensioactifs anioniques, les tensioactifs cationiques, les tensioactifs amphotères et les tensioactifs non-ioniques.

Les microémulsions de l'invention sont avantageusement utilisées pour la préparation de compositions appliquées à :
- la parfumerie fine, ou
- la cosmétique et les produits d'hygiène corporelle.
Ainsi, la présente invention se rapporte également à l'utilisation d'une microémulsion selon l'invention, pour la préparation d'une composition de parfumerie fine, ou d'une composition cosmétique ou d'hygiène corporelle.

Les microémulsions peuvent être utilisées en cosmétique. Elles peuvent alors contenir un ou plusieurs des composés choisis parmi des silicones, de l'huile paraffine, de l'isooctane, de l'isodécane, du squalène, du squalane, du sébum et de la lanoline.

Dans les exemples qui suivent, les structures et noms sont les suivants :

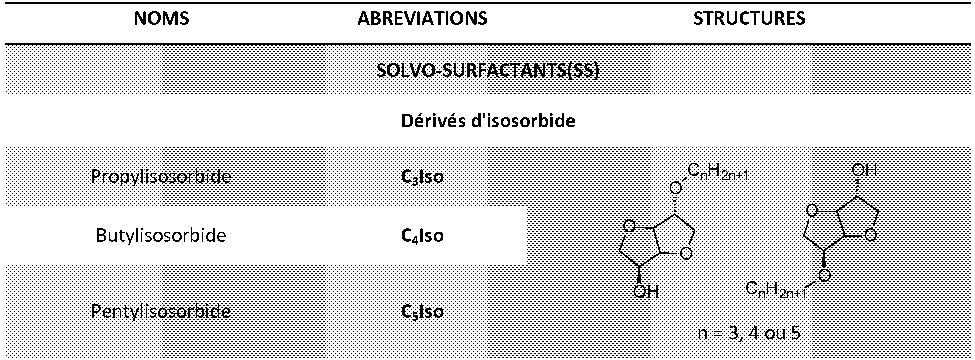

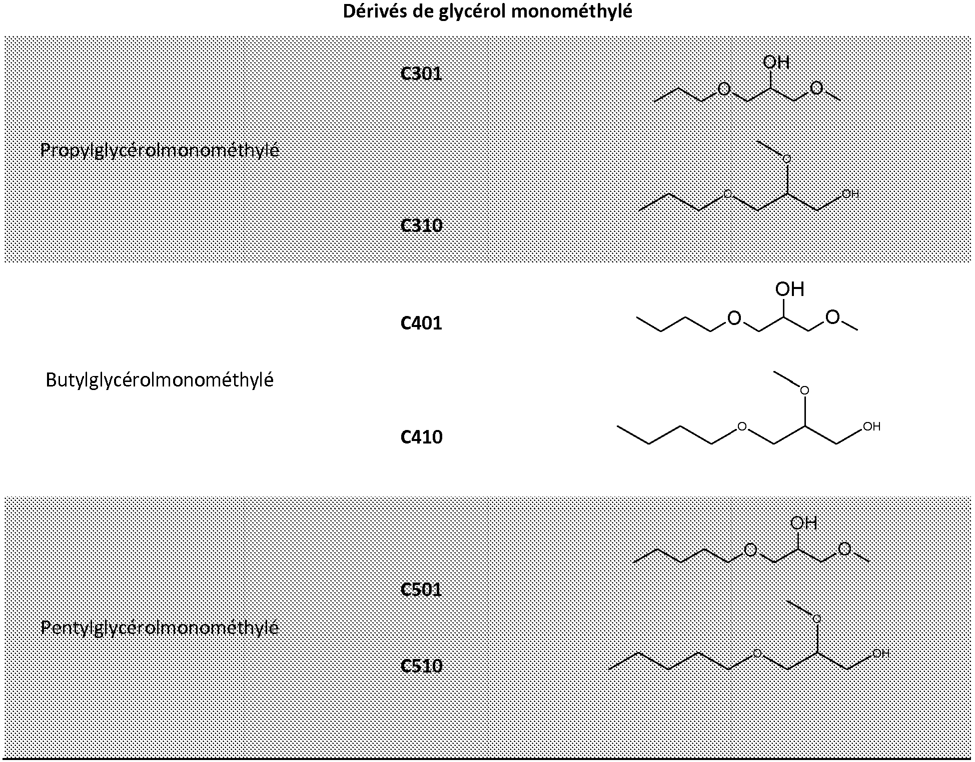

### Exemple 1 : Synthèse des solvo-surfactifs selon l'invention

Les synthèses du 1-méthoxy-3-propoxypropan-2-ol (C301) et du 1-méthoxy-3-pentoxypropan-2-ol (C501) se réalisent en deux étapes. La synthèse du 1-méthoxy-3-butoxypropan-2-ol (C401) est réalisée simplement par ouverture de l'époxyde dans la mesure où le réactif de départ, le butylglycidol, est accessible commercialement.

### I - Synthèse des dérivés de glycérol monométhylés:

### a) Condensation de l'alcool sur l'épichlorhydrine

La condensation de l'alcool (1 mole) sur l'épichlorhydrine se fait en léger excès d'épichlorhydrine (1,5 moles) en présence de ZnCl₂ comme catalyseur. L'épichlorhydrine est ajoutée goutte à goutte pendant 1h à 100°C. Le milieu réactionnel est ensuite maintenu à 115°C pendant 5h puis refroidi à 50°C. Un ajout goutte à goutte pendant 1h de NaOH (2,3 moles) à 48% est alors effectué. Toutes ces étapes de synthèse sont réalisées sous forte agitation. Une fois la réaction terminée, on ajoute de l'eau distillée et le produit est ainsi lavé 2 fois à l'eau pour éliminer les sels résiduels.

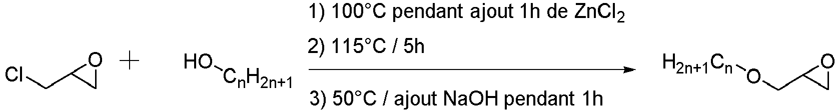

### Synthèse du Cₙglycidol, où n = 3, 5

Le produit obtenu est enfin distillé sous vide à 10-20 mbars entre 75 et 80°C.

### b) Ouverture de l'époxyde avec le méthanolate

Le Cₙglycidol (où n = 3, 4, 5) est ajouté goutte à goutte à une solution de méthanol contenant du méthanolate de sodium préalablement obtenu par réaction entre le méthanol et du sodium solide. L'ajout est effectué à reflux à 80°C pendant 30 min, puis la température de 80°C est maintenue pendant 24h jusqu'à disparition totale de Cₙglycidol :

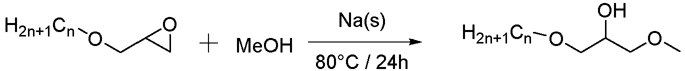

### Ouverture de l'époxyde par du méthanolate de sodium

### Purification :

Le méthanol est évaporé à l'évaporateur rotatif une fois la réaction terminée. Le produit obtenu est ensuite lavé avec deux solutions aqueuses saturées en NaCl : l'une contenant 3,4% de HCl et l'autre contenant 10% de NaHCO₃.

### II - Synthèse du C3Iso, et aussi des C4Iso et CSIso :

L'isosorbide est constitué de deux cycles présentant chacun une fonction hydroxyle, les fonctions étant non équivalentes en positions 2 et 5. Elles sont appelées respectivement positions *exo* et *endo.* La liaison hydrogène intramoléculaire de la fonction hydroxyle en position 5 et l'oxygène porté par le cycle adjacent discrimine les réactivités des deux positions ainsi que les propriétés des dérivés obtenus.

La réactivité des groupes hydroxyles est différente. Il est possible de jouer sur les paramètres de réaction (solvant, base, réactifs) pour obtenir différents ratios de dérivés d'isosorbide (monoéther *endo,* monoéther *exo* et diéther). Les solvants et bases utilisés pour réaliser l'alkylation sont respectivement le DMSO et le KOH. En effet, avec cette combinaison, le ratio *endo*/*exo* attendu sera de 1/1. Il est aussi possible d'utiliser du LiOH permettant d'obtenir majoritairement la forme *exo.* La réaction se fait en « one-pot » avec 1 équivalent de base, d'isosorbide et de bromoalkyle CₙBr. Tout d'abord, l'isosorbide est dissout dans le DMSO puis le mélange est chauffé à 90°C, le KOH est ensuite ajouté. Une fois ce dernier dissout totalement (1h), le CₙBr est ajouté goutte à goutte pendant 1h à reflux à 90°C. La réaction est arrêtée après 24h :

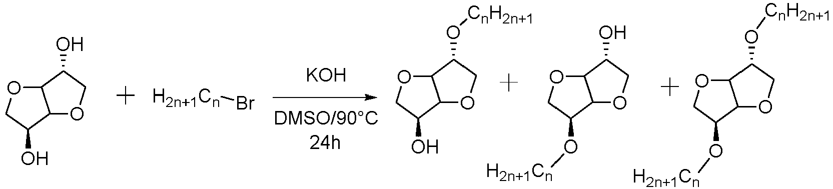

### Synthèse des monoalkyles d'isosorbide CₙIso avec n = 3, 4 ou 5

Trois produits sont obtenus : deux monoéthers (*endo* et *exo*) et le diéther.

### Purification :

Une fois la réaction terminée, il est nécessaire de rééquilibrer le pH de la solution. Pour cela, du NaHCO₃ est ajouté. Ensuite, pour éliminer un maximum de sel, une filtration sur fritté est effectuée avec deux lavages au DMSO. Le mélange réactionnel est ensuite distillé pour éliminer le DMSO de la solution. Le résidu de la distillation est ensuite solubilisé dans de l'acétate d'éthyle puis lavé deux fois avec de l'eau saturée en NaCl. L'acétate d'éthyle est ensuite évaporé à l'évaporateur rotatif pour récupérer les dérivés d'isosorbide présents dans la phase organique.

La séparation des mono- et diéthers est réalisée à l'aide d'une colonne de silice. Cette étape de séparation permet d'obtenir uniquement les deux monoéthers et d'éliminer le diéther et l'isosorbide restant. L'éluant choisi est un mélange acétate d'éthyle / éther de pétrole (20/80).

Il est possible de séparer les isomères *endo* et *exo* à l'aide d'une séparation par chromatographie par dépôt solide. Cette technique permet de rendre plus homogène l'élution des produits et donc de séparer plus facilement les deux isomères. Le produit est tout d'abord homogénéisé avec la silice à l'aide de dichlorométhane. Une fois le solvant évaporé à l'évaporateur rotatif, le solide contenant le produit est mis en tête de colonne d'une chromatographie classique. L'élution se fait ensuite de manière classique avec un éluant éther de pétrole / acétate d'éthyle (75/25). Une fois le premier isomère élué, la polarité de l'éluant est changée pour récupérer plus rapidement le deuxième isomère. L'éluant choisi est un mélange 50/50 éther de pétrole/acétate d'éthyle. On obtient ainsi les 2 isomères *endo* et *exo* séparément.

### Exemple 2 : Solubilisation de concentrés de parfums

### Solubilisation par les solvo-surfactifs seuls :

### Solubilisation de trois parfums et du linalol

Pour ces essais de référence dont le but était de classer les solvo-surfactifs selon leur capacité à solubiliser (ou non) les parfums, la quantité de parfum a été fixée à 5% massique dans l'eau milliQ. Les solubilisations des concentrés de parfums PF1, PF2 et PF3 ainsi que du linalol ont été évaluées.

Il apparaît que pour solubiliser les concentrés de parfums ou le linalol, il est nécessaire d'avoir une concentration en solvo-surfactif (SS) supérieure à 35% quelque soit le solvo-surfactif. Parmi tous les SS étudiés, le C₄Iso et le C301 semblent être les plus efficaces pour solubiliser seuls les fragrances.

### Solubilisation par les tensioactifs seuls :

Dans un second temps, la quantité minimale nécessaire de chaque tensioactif utilisé seul pour solubiliser 10% de parfum dans l'eau milliQ a été déterminée.

Les résultats montrent que le CrodaSinic LS30 (sodium lauroylsarcosinate) présente un mauvais pouvoir solubilisant des concentrés de parfums.

Les meilleures formulations pour les 3 concentrés de parfum sont :
- 11,5% de DTAB (tensioactif cationique) pour PF2 et PF3 ;
- 18% de SDS (tensioactif anionique) pour PF1.

En conclusion, sur l'ensemble des tests de solubilisation avec soit le solvo-surfactif seul, soit le tensioactif seul, on constate qu'il faut au minimum 35% de SS pour solubiliser 5% de parfum et 11-12% de tensioactif (TA) pour en solubiliser 10%.

### Solubilisation par combinaison solvo-surfactifs/tensioactifs :

Les combinaisons entre les SS, i.e.C₃Iso / C₄Iso / C301 / C401 / C501, et les TA, i.e. SDS / LES / DTAB / DHS / CAPB, ont été évaluées pour solubiliser 10% des concentrés de parfum PF1 à PF3.

### Solubilisation optimale

Les quantités optimales de SS et de TA nécessaires pour solubiliser chaque concentré ont été déterminées par ajouts successifs des deux composés jusqu'à l'obtention d'une phase limpide. On obtient ainsi la variation de la quantité de tensioactif à utiliser pour solubiliser les trois concentrés avec une quantité de SS fixée à 17%.

Pour une même concentration en SS, les quantités de TA nécessaires sont significativement différentes selon le concentré de parfum à solubiliser et le SS. De manière générale, on constate que les SDS, LES, DHS et DTAB sont plus efficaces pour réaliser une formulation limpide. Le TA amphotère CAPB donne les moins bons résultats. Par ailleurs, pour tous les concentrés, plus le nombre de carbones de la chaîne alkyle du SS est important, moins il est nécessaire d'utiliser de TA. Cette observation est contraire à la tendance observée lors de l'utilisation des solvo-surfactifs seuls. Ceci est dû à l'augmentation de l'interaction SS/TA lors de la solubilisation.

On peut aussi remarquer que la quantité de tensioactif nécessaire à la solubilisation varie suivant le concentré étudié.

Les formulations optimales pour chaque concentré sont les suivantes :

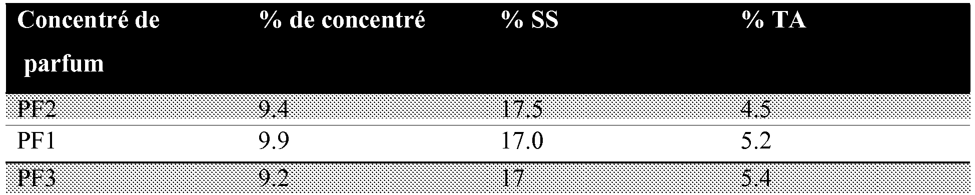

Chacune des formulations précédentes permet de solubiliser ≈ 10% de parfums. L'influence de la quantité de parfum à solubiliser sur la quantité de SDS nécessaire à la solubilisation du concentré PF2 a été étudiée en présence de 17% de C501. On constate que l'évolution du % de SDS augmente linéairement avec la quantité de parfum.

### Optimisation du ratio solvo-surfactif / tensioactif

Au cours des nombreux essais réalisés, il a été constaté que, selon le parfum, la quantité de tensioactif nécessaire dépend de la quantité de solvo-surfactif. Nous avons donc réalisé une étude un peu plus systématique de manière à déterminer les tendances et à affiner ce ratio, qui, par ailleurs, dépendra également de la stabilité de la formulation sur une gamme de température allant de 5 à 45°C. Les systèmes étudiés sont résumés dans le Tableau suivant :

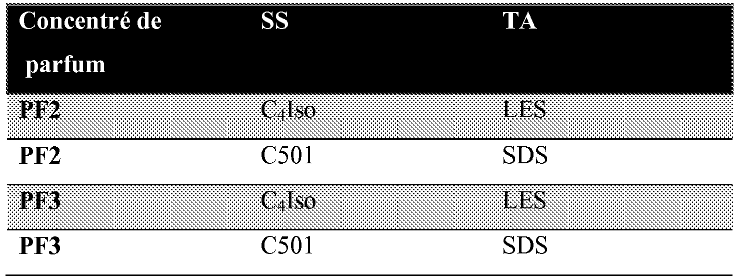

Les résultats obtenus pour chaque système montrent que les deux concentrés ont le même comportement suivant le couple de solvo-surfactif et tensioactif utilisé. Dans les deux cas, la diminution de la quantité de solvo-surfactif entraîne une augmentation de la quantité de tensioactif nécessaire à la solubilisation. Pour le couple C₄Iso/LES, la diminution de la quantité de C₄Iso induit une augmentation exponentielle du LES. Pour ce couple, il est préférable d'opter pour une formulation avec une concentration en solvo-surfactif supérieure à 20% pour éviter une utilisation trop élevée de tensioactif. Pour le couple C501/SDS, la diminution en solvo-surfactif n'induit qu'une faible augmentation de la quantité de SDS. Les formulations contenant peu de solvo-surfactif peuvent alors être utilisées pour solubiliser les concentrés car les quantités de tensioactifs correspondantes restent faibles. En conclusion, on constate qu'il n'y a pas d'évolution linéaire du ratio.

### Stabilité en température des formulations

Pour être acceptables, les formulations précédentes doivent être stables en température sur un intervalle de 5°C à 45°C ou préférentiellement de 15°C à 45°C. Les formulations sont donc placées dans un bain à 5°C et à 45°C, ou de 15°C à 45°C, pendant 24h. Après observation, les formulations limpides sont considérées comme stables et les formulations troubles sont considérées instables. Le tableau 1 ci-dessous reporte les compositions stables (« OK ») et les compositions ayant conduit à une déstabilisation (apparition d'un trouble) (« KO »).

**Tableau 1 : Stabilité des formulations contenant 10% de parfum à 5°C (ou 15°C), 25°C et 45°C**

| | **5°C** | **25°C** | **45°C** |
|---|---|---|---|
| **PF2:** | | | |
| C3Iso : 16.5-17.2% | 8.5% SDS : OK | 8.5% SDS : OK | 8.5% SDS : OK |
| | | 10% LES : OK | 10% LES : OK |
| C4Iso : 16-17.5% | 10% LES : OK | 5.3% SDS : OK | 5.3% SDS : OK |
| | | 3.1% LES : OK | 3.1% LES : OK |
| | 5.3% SDS : KO | | |
| | | | |
| | 3.1% LES : KO | | |

| **PF1:** | | | |
|---|---|---|---|
| C3Iso : 16.5% | 11.7% SDS : OK | 11.7% SDS : OK | 11.7% SDS : OK |
| | 12.6% LES : OK | 12.6% LES : OK | 12.6% LES : OK |
| C4Iso : 16.6% | | 8.2% SDS : OK | 8.2% SDS : OK |
| | 8.2% SDS : KO | 8.6% LES : OK | 8.6% LES : KO |
| | | | |
| | 8.6% LES : KO | | |

| **PF3:** | | | |
|---|---|---|---|
| C3Iso: 17% | 9.2% SDS : OK | 9.2% SDS : OK | 9.2% SDS : OK |
| | 10% LES : KO | 10% LES : OK | 10% LES : OK |
| C4Iso : 17-18% | 5.1% SDS : KO | 5.1% SDS : OK | 5.1% SDS : OK |
| | 4.3% LES : KO | 4.3% LES : OK | 4.3% LES : OK |
| | | | |

| | **15°C** | **25°C** | **45°C** |
|---|---|---|---|
| **PF2:** | | | |
| C301 : 16.5% | 8.4% SDS : OK | 8.4% SDS : OK | 8.4% SDS : OK |
| C401 : 17.5% | 5.4% SDS : OK | 5.4% SDS : OK | 5.4% SDS : KO |
| C501 : 17% | 4.6% SDS : OK | 4.6% SDS : OK | 4.6% SDS : KO |

| **PF1:** | | | |
|---|---|---|---|
| C301 : 16.4% | 11.2% SDS : OK | 11.2% SDS : OK | 11.2% SDS : OK |
| C401 : 17% | 7.2% SDS : OK | 7.2% SDS : OK | 7.2% SDS : OK |

| **PF3:** | | | |
|---|---|---|---|
| C301 : 16.5% | 8.8% SDS : OK | 8.8% SDS : OK | 8.8% SDS : OK |
| C401 : 17% | 6.7% SDS : OK | | |
| C501 : 13% | 4.7% SDS : OK | 6.7% SDS : OK | 6.7% SDS : OK |
| | | | |
| | | 4.7% SDS : OK | 4.7% SDS : KO |

| | | | |
|---|---|---|---|
| OK = stable ; KO = pas stable | | | |

Selon le solvo-surfactif utilisé, les stabilités aux différentes températures ne sont pas les mêmes. Le système C₃Iso/SDS montre une stabilité sur tout l'intervalle de température pour les 3 concentrés de parfum. Pour les autres solvo-surfactifs, les formulations ne sont pas stables à toutes les températures. A noter que, contrairement au C₄Iso, le C₃Iso comprend les formes endo et exo. Par ailleurs, les C401 et C501 possèdent un point de trouble traduisant la température à partir de laquelle le solvo-surfactif n'est plus soluble dans l'eau et s'agrège pour former un voile trouble dans l'eau. Ce point de trouble peut expliquer l'instabilité à 45°C des formulations utilisant le C501 et C401. Le point de trouble du C501 est d'environ 10°C, cependant les formulations restent limpides à température ambiante. Cette stabilité à une température supérieure à celle du point de trouble du solvo-surfactif s'explique par l'augmentation du point de trouble du C501 lors de l'ajout du SDS.

L'effet sur le point de trouble des C401 et C501, de l'urée, du dipropylène glycol (DPG) et SDS a été testé. Les résultats montrent qu'il est possible d'augmenter le point de trouble des deux solvo-surfactifs par ajout d'urée et de DPG. Cette augmentation reste toutefois beaucoup moins importante que celle observée lors de l'ajout de SDS.

Au vu de ces résultats, des formulations contenant un mélange de 2 solvo-surfactifs ont été préparées : l'un donnant une bonne stabilité en température et l'autre une bonne efficacité à la solubilisation, dans le but de voir s'il est ainsi possible d'augmenter l'intervalle de stabilité en température tout en augmentant l'efficacité de solubilisation. Le C501 a été retenu comme ayant une bonne efficacité et le C₃Iso permet d'obtenir une bonne stabilité en température. Les 2 solvo-surfactifs ont été mélangés à différents ratios et pour chaque mélange, la formulation optimale de solubilisation en présence de SDS a été déterminée. Pour chaque formulation, 10% de PF2 ont été solubilisés.

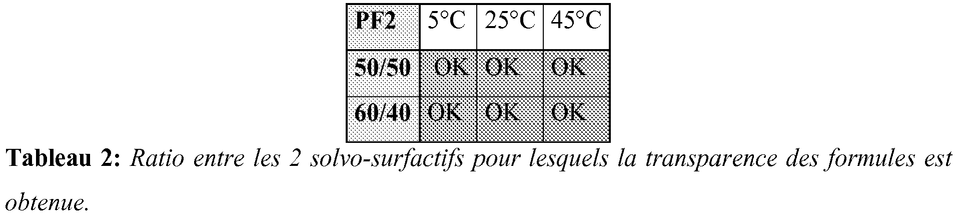

Les concentrations en SDS nécessaires pour solubiliser les parfums dans l'eau sont similaires à celles nécessaires lorsque le C501 est utilisé seul (≈ 5-6%). On obtient une bonne stabilité en température de 5 à 45°C pour plusieurs ratios C₃Iso/C501.

L'effet synergique entre les solvo-surfactifs et les tensioactifs a ensuite été démontré par une diminution conséquente de la quantité de matière active dans les formulations à base des systèmes solvo-surfactifs/tensioactifs par rapport aux formulations avec les tensioactifs ou solvo-surfactifs seuls.

### Exemple 3 : Solubilisation de concentrés de parfums

### 1) Tests avec C501 et C510 :

On prépare les compositions suivantes :

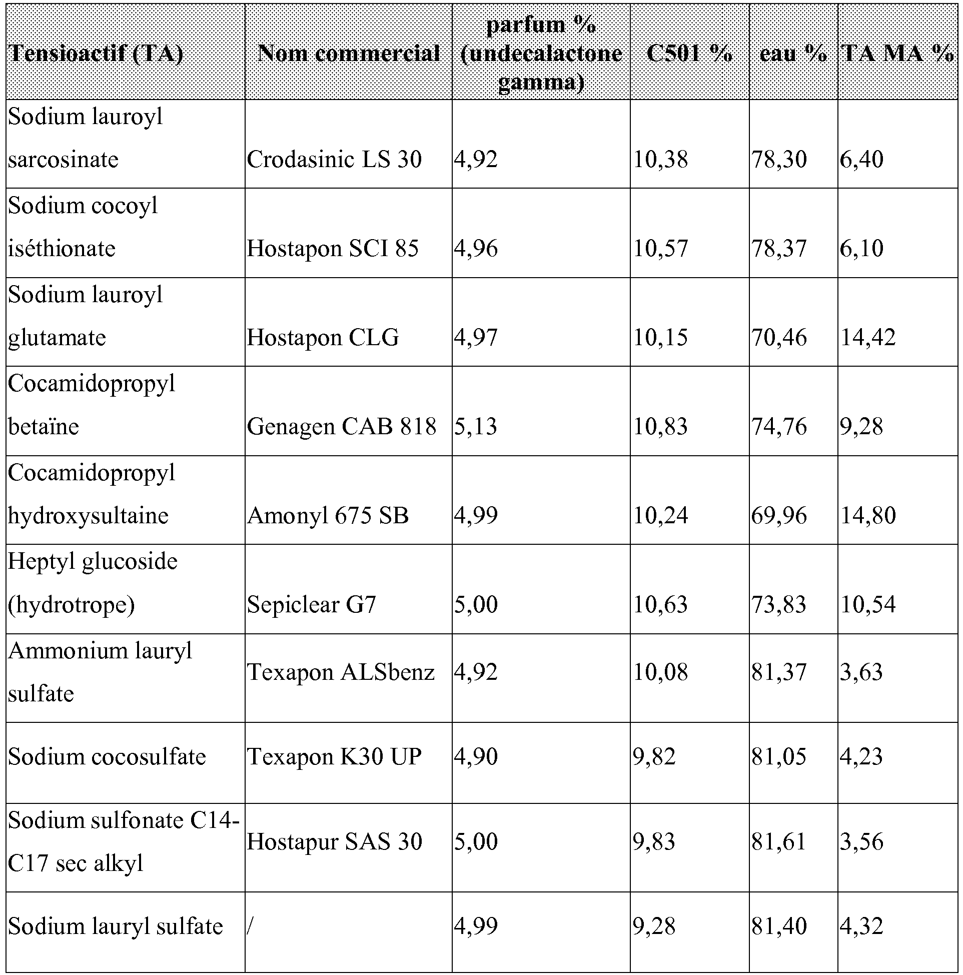

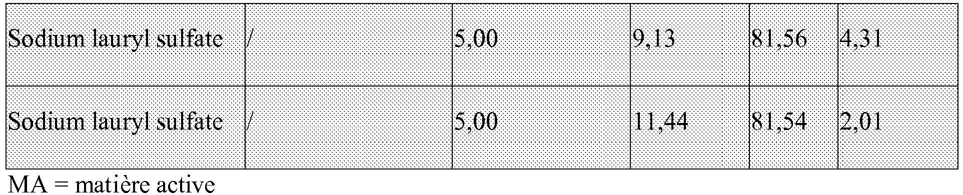

On observe que toutes les compositions préparées sont transparentes et stables.

### 2) Tests avec C510 et différents parfums :

Les parfums A, B et C suivants sont préparés :

| Parfum A | % |
|---|---|
| Undecalactone gamma | 20 |
| Methyl ionone gamma | 20 |
| Ionone beta | 20 |
| Cis 3 hexenyl acetate | 20 |
| Iso gamma super | 20 |

| Parfum B | % |
|---|---|
| Hélional | 20 |
| Triplai | 20 |
| Hexyl cinnamique aldehyde | 20 |
| Cis 3 hexenyl benzoate | 20 |
| Eugenol | 20 |

| Parfum C | % |
|---|---|
| Hélional | 20 |
| Methyl ionone gamma | 20 |
| Ionone beta | 20 |
| Cis 3 hexenyl acetate | 20 |
| Iso gamma super | 20 |

Puis les compositions suivantes sont préparées :

| **Nom du parfum** | **Parfum %** | **C510 %** | **Eau %** | **SDS MA %** |
|---|---|---|---|---|
| PA | 4,96 | 10,39 | 82,64 | 2,01 |
| PB | 4,95 | 10,03 | 79,21 | 5,81 |
| PC | 5,00 | 9,95 | 80,03 | 5,02 |

SDS MA = sodium dodécyl sulfate, matière active

On observe que toutes les compositions préparées sont transparentes et stables.

### Exemple 4 : Formulations de parfums

Le jus de parfum suivant (P) a été préparé :

| Nom du parfum | % | CAS # |
|---|---|---|
| UNDECALACTONE GAMMA | 0.5 | 104-67-6 |
| CIS 3 HEXENYL ACETATE | 1.0 | 3681-71-8 |
| HEXYLCINNAMIQUE ALDEHYDE | 12.0 | 101-86-0 |
| HEXENYLE CIS 3 BENZOATE | 2.0 | 25152-85-6 |
| EUGENOL | 2.0 | 97-53-0 |
| HELIONAL | 5.0 | 1205-17-0 |
| IONONE BETA | 3.0 | 14901-07-6 |
| METHYLIONONE GAMMA | 5.0 | 127-51-5 |
| BENZYL PROPIONATE | 1.0 | 122-63-4 |
| TRIPLAL | 0.5 | 68039-49-6 |
| BENZYLE ACETATE | 5.0 | 140-11-4 |
| HEDIONE HC | 30.0 | 24851-98-7 |
| ISO GAMMA SUPER | 26.0 | 68155-66-8 |
| Cis 3 HEXENYLE SALICYLATE | 5.0 | 65405-77-8 |
| VANILINE | 2.0 | 121-33-5 |

Puis les formulations suivantes ont été préparées :

| Nature du solvo-surfactant | Solvo-Surfactant | Parfum | Tensio-actif LES | Eau |
|---|---|---|---|---|
| C410 | 14.5% | 5% | 5% | 72.4% |
| C401 | 20% | 5% | 2.6% | 78.5% |

A titre comparatif, il faut 17.6% de LES seul pour solubiliser 2.9% de P.

Il ressort ainsi que C401 et C410, mais surtout C410, sont de bons solvo-surfactifs pour solubiliser P, en combinaison avec le lauryléther (laureth) sulfate de sodium (LES).

| Nature du solvo-surfactant | Solvo-Surfactant | Parfum | Tensio-actif LES | Eau |
|---|---|---|---|---|
| C510 | 14% | 5% | 2.5% | 78.5% |
| C501 | 14.5% | 5% | 5% | 75.5% |

C501 et C510 sont donc de bons solvo-surfactifs pour solubiliser P, en combinaison avec le lauryléther (laureth) sulfate de sodium (LES).

## Revendications

1. Microémulsion de type huile-dans-eau comprenant, de préférence consistant en, en poids par rapport au poids total de microémulsion:
• 70% à 94% d'eau,
• 1% à 15% d'au moins une substance hydrophobe parfumante,
• 4% à 20% d'au moins un solvo-surfactif de préférence volatil, et
• 0,1% à 15%, de préférence 1% à 13%, d'au moins un agent hydrotrope ou d'au moins un tensioactif choisi parmi les tensioactifs anioniques, les tensioactifs cationiques, les tensioactifs amphotères et les tensioactifs non ioniques,
ledit solvo-surfactif étant choisi parmi les dérivés de glycérol monoalkylé de formule (I) suivante : 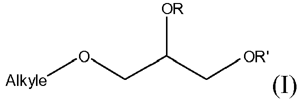 dans lesquels le groupe « Alkyle » est un groupe alkyle linéaire ou ramifié comprenant de 1 à 8 atomes de carbone, et R et R' sont chacun indépendamment H ou un groupe alkyle linéaire ou ramifié comprenant de 1 à 5 atomes de carbone, à la condition que R est différent de R', et leurs mélanges.

2. Microémulsion selon la revendication 1, dans laquelle la substance hydrophobe parfumante est une substance hydrophobe parfumante naturelle choisie parmi les terpènes, les huiles essentielles et les composés naturels présentant des propriétés odoriférantes, notamment choisis parmi les aldéhydes, les esters, les cétones, les alcools, les phénols, les alcènes et les éthers.

3. Microémulsion selon la revendication 1 ou 2, dans laquelle dans le dérivé de glycérol monoalkylé de formule (I), le groupe « Alkyle » est un groupe alkyle linéaire comprenant 3, 4 ou 5 atomes de carbone, et R et R' sont chacun indépendamment H ou un groupe méthyle ou éthyle, à la condition que R est différent de R'.

4. Microémulsion selon l'une des revendications 1 à 3, dans laquelle dans le dérivé de glycérol monoalkylé de formule (I) le groupe « Alkyle » est un groupe alkyle linéaire comprenant 3, 4 ou 5 atomes de carbone, R est un groupe méthyle et R' est H.

5. Microémulsion selon l'une des revendications 1 à 4, qui est substantiellement exempte d'éthanol, de préférence qui est dépourvue d'éthanol.

6. Microémulsion selon l'une des revendications 1 à 5, consistant en :
• 70% à 90% d'eau,
• 5% à 12% d'au moins une substance hydrophobe parfumante,
• 4% à 18% d'au moins un solvo-surfactif volatil, et
• 0,1% à 10% d'au moins un agent hydrotrope ou d'au moins un tensioactif choisi parmi les tensioactifs anioniques, les tensioactifs cationiques, les tensioactifs amphotères et les tensioactifs non-ioniques.

7. Microémulsion selon l'une des revendications 1 à 6, dans laquelle le tensioactif est un tensioactif anionique choisi parmi :
• les alkylsulfonates, notamment le sulfosuccinate de dihexyle de formule 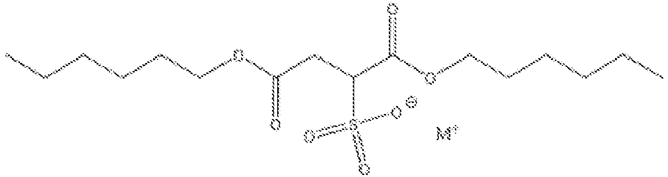 dans laquelle M⁺ représente Na⁺, K⁺, NH₄⁺, (HOCH₂CH₂)₃NH⁺,
ou notamment le sulfosuccinate d'éthyl-2-hexyle de formule 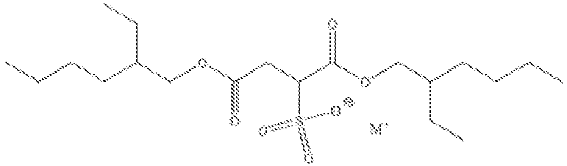 dans laquelle M⁺ représente Na⁺, K⁺, NH₄⁺, (HOCH₂CH₂)₃NH⁺,
• les alkylarylsulfonates de formule 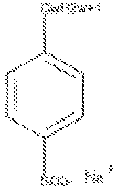 dans laquelle w est un entier compris de 8 à 12, en particulier l'isooctyl, l'isononyl et notamment l'isododécylbenzènesulfonate de sodium de formule 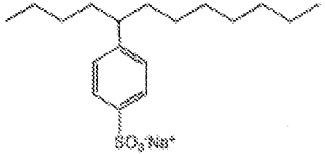
• les propoxysulfates de formule 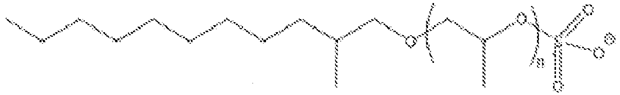 dans laquelle le nombre de motifs propoxylate n est compris de 4 à 8,
• les alkylsulfates, notamment les sels du sulfate de lauryle tel que le dodécyl sulfate de sodium et les alkyléther sulfates de sodium tel que le lauryléther (laureth) sulfate de sodium,
• et les sels d'acides gras de formule R-CO₂⁻ M⁺, dans laquelle R représente une chaîne carbonée, linéaire ou ramifiée, saturée ou insaturée, contenant 8 à 18 atomes de carbone, et M⁺ représente un cation choisi parmi les ions Na⁺, K⁺, NH₄⁺, (HOCH₂CH₂)₃NH⁺, notamment le sel d'acide oléique de formule CH₃(CH2)₇CH=CH(CH₂)₇CO₂⁻ M⁺ dans lequel M⁺ a les significations définies ci-dessus.

8. Microémulsion selon l'une des revendications 1 à 6, dans laquelle le tensioactif est un tensioactif non ionique choisi parmi :
• les alcanolamides, notamment le monoéthanolamide de formule 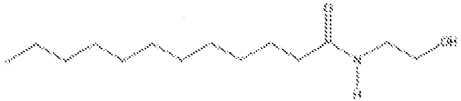 sous réserve que ledit alcanolamide ne soit pas un polyol,
• les alkylpolyglycosides,
• les éthers de polyglycérol,
• et les esters de polyglycérol, ou bien
le tensioactif est un tensioactif amphotère choisi parmi:
• les bétaïnes, notamment la bétaïne de cocamidopropyle de formule 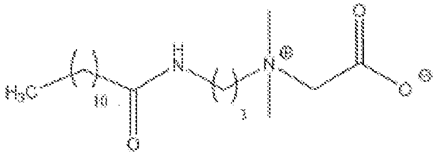
• et les oxydes d'amine, notamment l'oxyde de lauryldiméthylamine de formule 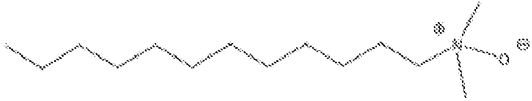 ou bien
le tensioactif est un tensioactif cationique choisi parmi:
• des sels de tétraalkylammonium, notamment des sels de dialkyldiméthylammonium de formule 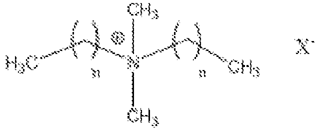 dans laquelle
X⁻ représente un anion choisi parmi Cl⁻ , Br⁻, I⁻, CH₃COO⁻ ou lactate,
n est le nombre de méthylène compris de 6 à 12,
de préférence un sel de didécyldiméthylammonium de formule 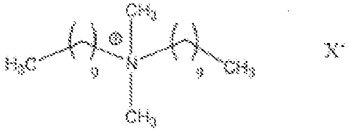 dans laquelle X⁻ a les significations désignées ci-dessus,
• des sels de trialkylammonium, en particulier des halogénures de trialkylammonium, notamment le bromure de dodécyltriméthylammoniumde formule H₃C(CH₂)ₙN⁺(CH3)3,
• des sels de pyridinium, notamment le sel de cétylpyridinium de formule 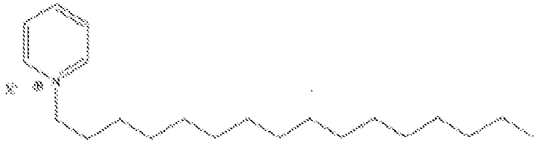 dans laquelle X⁻ a les significations désignées ci-dessus,
• des sels de benzalkonium, notamment des sels de formule 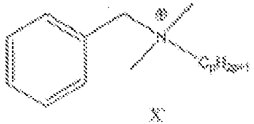 dans laquelle
X⁻ a les significations désignées ci-dessus,
p est compris de 8 à 18,
• des sels d'ammonium de la triéthanolamine de formule
(HO-CH₂-CH₂)₃NH⁺ X- dans laquelle X⁻ a les significations désignées ci-dessus.

9. Microémulsion selon l'une des revendications 1 à 6, dans laquelle l'agent hydrotrope est choisi parmi les acides aryl sulfoniques et les alkyl glucosides, de préférence il est l'heptyl glucoside.

10. Utilisation d'une microémulsion selon l'une des revendications 1 à 9, pour la préparation d'une composition de parfumerie fine, ou d'une composition cosmétique ou d'hygiène corporelle.

## Patentansprüche

1. Mikroemulsion vom Öl-in-Wasser-Typ, umfassend, vorzugsweise bestehend aus, als Gewicht relativ zum Gesamtgewicht der Mikroemulsion:
• 70 % bis 94 % Wasser,
• 1 % bis 15 % wenigstens einer hydrophoben Parfümsubstanz,
• 4 % bis 20 % wenigstens eines vorzugsweise flüchtigen Solvat-Tensids, und
• 0,1 % bis 15 %, vorzugsweise 1 % bis 13 %, wenigstens eines hydrotropen Mittels oder wenigstens eines Tensids, gewählt aus nichtionischen Tensiden, kationischen Tensiden, amphoteren Tensiden und nichtionischen Tensiden,
wobei das Solvat-Tensid gewählt ist aus Monoalkylglycerinderivaten der folgenden Formel (I): 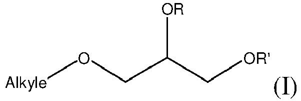 wobei die "Alkyl"-Gruppe eine lineare oder verzweigte Alkylgruppe, umfassend 1 bis 8 Kohlenstoffatome, ist, und R und R' jeweils unabhängig voneinander H oder eine lineare oder verzweigte Alkylgruppe, umfassend 1 bis 5 Kohlenstoffatome, sind, mit der Maßgabe, dass R von R' verschieden ist, sowie deren Mischungen.

2. Mikroemulsion nach Anspruch 1, wobei die hydrophobe Parfümsubstanz eine natürliche hydrophobe Parfümsubstanz ist, gewählt aus Terpenen, ätherischen Ölen und natürlichen Verbindungen mit duftenden Eigenschaften, insbesondere gewählt aus Aldehyden, Estern, Ketonen, Alkoholen, Phenolen, Alkenen und Ethern.

3. Mikroemulsion nach Anspruch 1 oder 2, wobei in dem monoalkylierten Glycerinderivat der Formel (I) die "Alkyl"-Gruppe eine lineare Alkylgruppe, umfassend 3, 4 oder 5 Kohlenstoffatome, ist und R und R' jeweils unabhängig voneinander H oder eine Methyl- oder Ethylgruppe sind, mit der Maßgabe, dass R von R' verschieden ist.

4. Mikroemulsion nach einem der Ansprüche 1 bis 3, wobei in dem monoalkylierten Glycerinderivat der Formel (I) die "Alkyl"-Gruppe eine lineare Alkylgruppe mit 3, 4 oder 5 Kohlenstoffatomen ist, R eine Methylgruppe ist und R' H ist.

5. Mikroemulsion nach einem der Ansprüche 1 bis 4, die im Wesentlichen frei von Ethanol, vorzugsweise ganz frei von Ethanol ist.

6. Mikroemulsion nach einem der Ansprüche 1 bis 5, umfassend :
• 70 % bis 90 % Wasser,
• 5 % bis 12 % wenigstens einer hydrophoben Parfümsubstanz,
• 4 % bis 18 % wenigstens eines flüchtigen Solvat-Tensids, und
• 0,1 % bis 10 % wenigstens eines hydrotropen Mittels oder wenigstens eines Tensids, gewählt aus nichtionischen Tensiden, kationischen Tensiden, amphoteren Tensiden und nichtionischen Tensiden.

7. Mikroemulsion nach einem der Ansprüche 1 bis 6, wobei das Tensid ein nichtionisches Tensid ist, gewählt aus:
• Alkylsulfonaten, insbesondere Dihexylsulfosuccinat der Formel 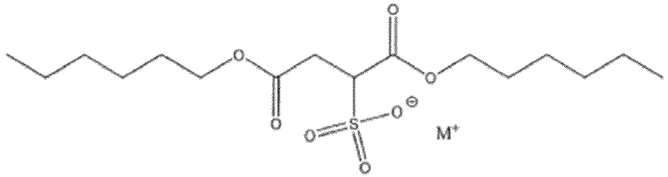 wobei M⁺ für Na⁺, K⁺, NH₄⁺, (HOCH₂CH₂)₃NH+ steht,
oder insbesondere für Ethyl-2-hexylsulfosuccinat der Formel 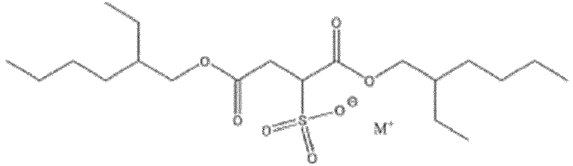 wobei M⁺ für Na⁺, K⁺, NH₄⁺, (HOCH₂CH₂)₃NH⁺ steht,
• Alkylarylsulfonaten der Formel 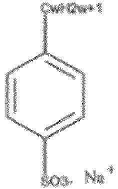 wobei w eine ganze Zahl zwischen 8 und 12 ist, insbesondere Isooctyl, Isononyl und insbesondere Natriumisododecylbenzolsulfonat der Formel 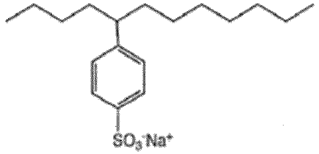
• Propoxysulfaten der Formel 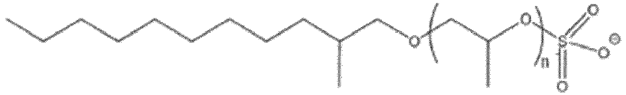 wobei die Anzahl der Propoxylateinheiten n 4 bis 8 beträgt,
• Alkylsulfaten, einschließlich Salzen von Laurylsulfat wie Natriumdodecylsulfat und Natriumalkylethersulfat wie Natriumlaurylsulfat (Laureth),
• und Salzen von Fettsäuren der Formel R-CO₂⁻M⁺, wobei R für eine lineare oder verzweigte, gesättigte oder ungesättigte Kohlenstoffkette mit 8 bis 18 Kohlenstoffatomen steht und M⁺ ein Kation darstellt, gewählt aus den Ionen Na⁺, K⁺, NH₄⁺, (HOCH₂CH₂)₃NH⁺, insbesondere das Ölsäuresalz der Formel CH₃(CH2)₇CH=CH(CH₂)₇CO₂⁻ M⁺ wobei M⁺ die oben definierten Bedeutungen hat.

8. Mikroemulsion nach einem der Ansprüche 1 bis 6, wobei das Tensid ein nichtionisches Tensid ist, gewählt aus:
• Alkanolamiden, insbesondere Monoethanolamid der Formel 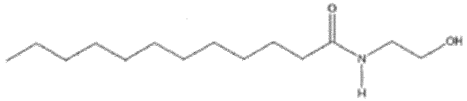 mit der Maßgabe, dass das Alkanolamid kein Polyol ist,
• Alkylpolyglycosiden,
• Polyglycerin-Ethern,
• und Polyglycerinestern, oder
das Tensid ein amphoteres Tensid ist, gewählt aus:
• Betainen, insbesondere Kokamidopropylbetain der Formel 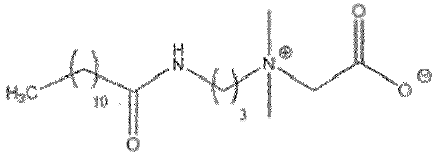
• und Aminoxiden, insbesondere Lauryldimethylaminoxid der Formel 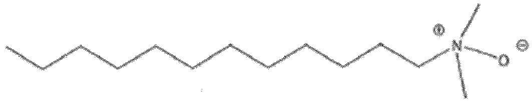 oder
das Tensid ein kationisches Tensid ist, gewählt aus:
• Tetraalkylammoniumsalzen, insbesondere Dialkyldimethylammoniumsalzen der Formel 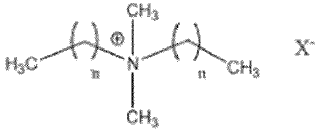 wobei
X⁻ für ein Anion steht, gewählt aus Cl⁻ , Br⁻, I⁻, CH₃COO⁻ oder Lactat,
n für die Methylenzahl von 6 bis 12 steht,
vorzugsweise ein Didecyldimethylammoniumsalz der Formel 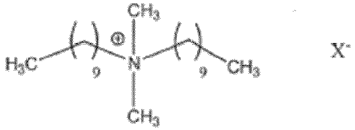 wobei X⁻ die oben angegebenen Bedeutungen hat,
• Trialkylammoniumsalzen, insbesondere Trialkylammoniumhalogeniden, insbesondere Dodecyltrimethylammoniumbromid der Formel H₃C(CH₂)₁₁N⁺(CH3)3,
• Pyridiniumsalzen, insbesondere Cetylpyridiniumsalz der Formel 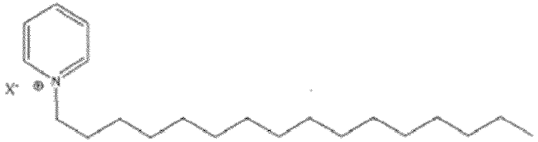 wobei X⁻ die oben angegebenen Bedeutungen hat,
• Benzalkoniumsalzen, insbesondere Salzen der Formel 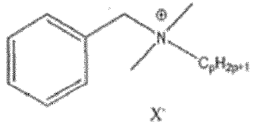 wobei
X⁻ die oben angegebenen Bedeutungen hat,
p zwischen 8 und 18 liegt,
• Ammoniumsalzen von Triethanolamin der Formel (HO-CH₂-CH₂)₃NH⁺ X-, wobei X⁻ die oben angegebenen Bedeutungen hat.

9. Mikroemulsion nach einem der Ansprüche 1 bis 6, wobei das hydrotrope Mittel gewählt ist aus Arylsulfonsäuren und Alkylglucosiden und vorzugsweise Heptylglucosid ist.

10. Verwendung einer Mikroemulsion nach einem der Ansprüche 1 bis 9 zur Herstellung einer Zusammensetzung der feinen Parfümerie oder einer kosmetischen oder Körperpflege-Zusammensetzung.

## Claims

1. A microemulsion of oil-in-water type comprising, preferably consisting of, by weight relative to the total weight of microemulsion:
• 70% to 94% of water,
• 1% to 15% of at least one hydrophobic fragrancing substance,
• 4% to 20% of at least one preferably volatile solvo-surfactant, and
• 0.1% to 15%, preferably 1% to 13%, of at least one hydrotropic agent or at least one surfactant selected from anionic surfactants, cationic surfactants, amphoteric surfactants and non-ionic surfactants,
said solvo-surfactant being selected from monoalkylated glycerol derivatives of following formula (I): 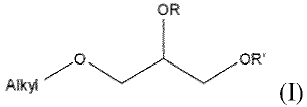 wherein the "alkyl" group is a linear or branched alkyl group comprising from 1 to 8 carbon atoms, and R and R' are each independently H or a linear or branched alkyl group comprising from 1 to 5 carbon atoms, with the proviso that R is different from R', and mixtures thereof.

2. The microemulsion as claimed in claim 1, wherein the hydrophobic fragrancing substance is a natural hydrophobic fragrancing substance selected from terpenes, essential oils and natural compounds having odoriferous properties, especially selected from aldehydes, esters, ketones, alcohols, phenols, alkenes and ethers.

3. The microemulsion as claimed in claim 1 or 2, wherein, in the monoalkylated glycerol derivative of formula (I), the "alkyl" group is a linear alkyl group comprising 3, 4 or 5 carbon atoms, and R and R' are each independently H or a methyl or ethyl group, with the proviso that R is different from R'.

4. The microemulsion as claimed in one of claims 1 to 3, wherein, in the monoalkylated glycerol derivative of formula (I), the "alkyl" group is a linear alkyl group comprising 3, 4 or 5 carbon atoms, and R is a methyl group, and R' is H.

5. The microemulsion as claimed in one of claims 1 to 4, which is substantially free of ethanol and which is preferably devoid of ethanol.

6. The microemulsion as claimed in one of claims 1 to 5, consisting of:
• 70% to 90% of water,
• 5% to 12% of at least one hydrophobic fragrancing substance,
• 4% to 18% of at least one volatile solvo-surfactant, and
• 0.1% to 10% of at least one hydrotropic agent or at least one surfactant selected from anionic surfactants, cationic surfactants, amphoteric surfactants and non-ionic surfactants.

7. The microemulsion as claimed in one of claims 1 to 6, wherein the surfactant is an anionic surfactant selected from:
• alkyl sulfonates, especially dihexyl sulfosuccinate of formula 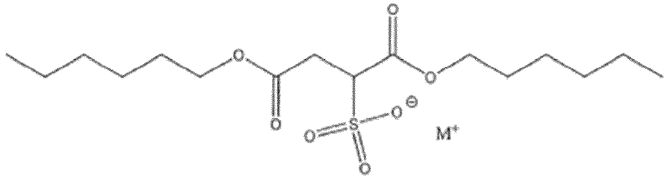 wherein M⁺ represents Na⁺, K⁺, NH₄⁺, (HOCH₂CH₂)₃NH⁺,
or especially 2-ethylhexyl sulfosuccinate of formula 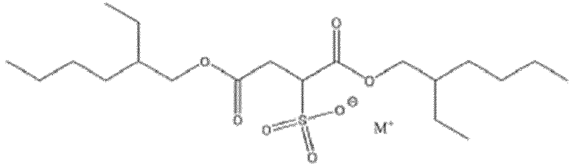 wherein M⁺ represents Na⁺, K⁺, NH₄⁺, (HOCH₂CH₂)₃NH⁺,
• alkylaryl sulfonates of formula 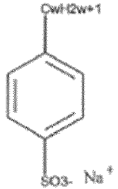 wherein w is an integer from 8 to 12, in particular sodium isooctyl-, isononyl- and especially isododecylbenzenesulfonate of formula 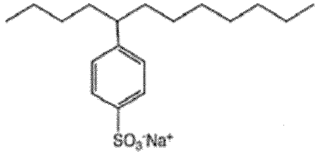
• propoxy sulfates of formula 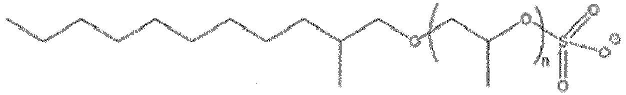 wherein the number of propoxylate units n is from 4 to 8,
• alkyl sulfates, especially salts of lauryl sulfate such as sodium dodecyl sulfate and sodium alkylether sulfates such as sodium lauryl ether (laureth) sulfate,
• and salts of fatty acids of formula R-CO₂⁻M⁺, wherein R represents a linear or branched, saturated or unsaturated carbon-based chain containing 8 to 18 carbon atoms, and M⁺ represents a cation selected from the ions Na⁺, K⁺, NH₄⁺, (HOCH₂CH₂)₃NH⁺, especially the oleic acid salt of formula CH₃(CH₂)₇CH=CH(CH₂)₇CO₂⁻ M⁺, wherein M⁺ has the above-defined meanings.

8. The microemulsion as claimed in one of claims 1 to 6, wherein the surfactant is a non-ionic surfactant selected from:
• alkanolamides, especially the monoethanolamide of formula 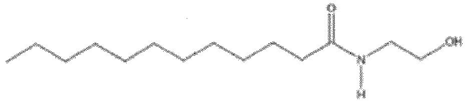 with the proviso that said alkanolamide is not a polyol,
• alkylpolyglycosides,
• polyglycerol ethers,
• and polyglycerol esters, or else
the surfactant is an amphoteric surfactant selected from:
• betaines, especially cocamidopropyl betaine of formula 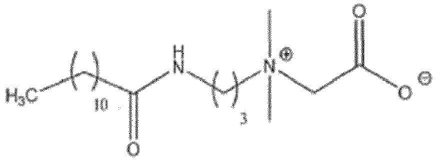
• and amine oxides, especially lauryldimethylamine oxide of formula 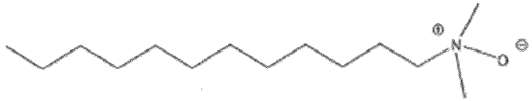 or else
the surfactant is a cationic surfactant selected from:
• tetraalkylammonium salts, especially dialkyldimethylammonium salts of formula 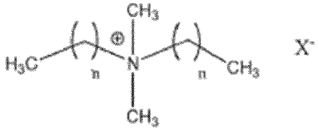 wherein
X⁻ represents an anion selected from Cl⁻, Br⁻, I⁻, CH₃COO⁻ or lactate,
n is the number of methylenes, from 6 to 12,
preferably a didecyldimethylammonium salt of formula 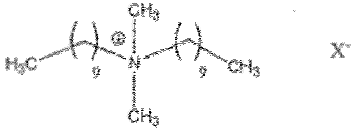 wherein X⁻ has the meanings given above,
• trialkylammonium salts, in particular trialkylammonium halides, especially dodecyltrimethylammonium bromide of formula H₃C(CH₂)₁₁N⁺(CH₃)₃,
• pyridinium salts, especially cetylpyridinium salt of formula 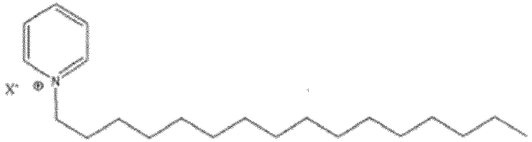 wherein X⁻ has the meanings given above,
• benzalkonium salts, especially salts of formula 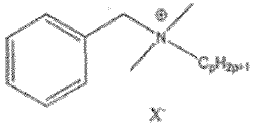 wherein
X⁻ has the meanings given above,
p is from 8 to 18,
• ammonium salts of triethanolamine of formula (HO-CH₂-CH₂)₃NH⁺ X- wherein X⁻ has the meanings given above.

9. The microemulsion as claimed in one of claims 1 to 6, wherein the hydrotropic agent is selected from arylsulfonic acids and alkyl glucosides; it is preferably heptyl glucoside.

10. The use of a microemulsion as claimed in one of claims 1 to 9 for the preparation of a fine fragrance composition or of a cosmetic or body hygiene composition.
